# EUROPEAN PATENT APPLICATION

(11) **EP 4 209 556 A1**
(43) Date of publication of application: **12.07.2023**
(21) Application number: 21864185.0
(22) Date of filing: 24.08.2021
(51) Int. Cl.: C09D 11/328, A61K 9/44

(54) **INKJET INK AND TABLET**

(30) Priority: 04.09.2020 JP 2020149379
(71) Applicant: TOPPAN INC., Tokyo 110-0016 (JP)
(72) Inventor: KOSEKI, Seiya, Tokyo 110-0016 (JP); ISHIKAWA, Hideki, Tokyo 110-0016 (JP); HOSHINO, Yuichi, Tokyo 110-0016 (JP); SAITO, Masatoshi, Tokyo 110-0016 (JP)
(74) Representative: TBK
(86) International application number: PCT/JP2021/030983
(87) International publication number: WO 2022/050127

(57) **Abstract**

The present invention aims to provide an inkjet ink which can sufficiently suppress photofading of printed images and has good lightfastness, and to provide a tablet including a printed part that is printed using the inkjet ink. The inkjet ink contains at least one of Red No. 102 and Blue No. 1 as a food dye, and contains a disaccharide whose solubility in 100 ml of water at 20°C is less than 20 g, as a fixing agent.

## Description

### [Technical Field]

The present invention relates to inkjet inks and tablets.

### [Background Art]

Inkjet printing inks (which may also be simply termed inkjet ink(s) hereinafter) include those inks which are edible due to use of food dyes, such as tar pigments, as colorants.

Inkjet inks based on the conventional art may cause discoloration, i.e., change of color (hue) of colorants in printed characters, images, and the like due to deterioration of the colorants, or may cause fading due to desaturation or the like of the colorants. For example, if inkjet inks are insufficient in lightfastness, the colorants may be affected by light and decompose (undergo photolysis) to cause discoloration (photofading). Such discoloration is perceived as visibility deterioration in printed characters, images, and the like (which are collectively referred to as printed image(s) hereinafter).

To suppress discoloration of such printed images, technologies are being developed, and various methods to suppress discoloration have been proposed (e.g., see PTL 1). However, methods based on the conventional art have been insufficient for suppressing photofading of printed images, and thus lightfastness of inkjet inks has not been improved.

### [Citation List]

### [Patent Literature]

PTL 1: JP 6389506 B

### [Summary of the Invention]

### [Technical Problem]

The present invention has been made in light of the circumstances described above and aims to provide an inkjet ink which can sufficiently suppress photofading of printed images and improve lightfastness, and to provide a tablet including a printed part that is printed using the inkjet ink.

### [Solution to Problem]

In order to accomplish the above aim, an inkjet ink according to an aspect of the present invention contains at least one of Red No. 102 and Blue No. 1 as a food dye; and a disaccharide whose solubility in 100 ml of water at 20°C is less than 20 g, as a fixing agent.

Furthermore, in order to accomplish the above aim, a tablet according to an aspect of the present invention includes a printed part that is printed using the ink jet ink.

### [Advantageous Effects of the Invention]

According to an aspect of the present invention, photofading of printed images can be sufficiently suppressed and lightfastness of the inkjet ink can be improved.

### [Brief Description of the Drawings]

Fig. 1 is a set of diagrams illustrating permeability and lightfastness of an inkjet ink according to an embodiment of the present invention.
Fig. 2 is a schematic cross-sectional view illustrating an example of a tablet (uncoated tablet) according to an embodiment of the present invention.
Fig. 3 is a schematic cross-sectional view illustrating an example of a tablet (film-coated tablet) according to an embodiment of the present invention.
Fig. 4 is a diagram illustrating an example of a printed image on a tablet (uncoated tablet) according to an embodiment of the present invention.
Fig. 5 is a diagram illustrating an example of a printed image on a tablet (film-coated tablet) according to an embodiment of the present invention.

### [Description of the Embodiments]

An inkjet ink according to an embodiment of the present invention (termed the present embodiment hereinafter) relates to inkjet inks which can sufficiently suppress photofading and improve lightfastness of printed images provided on, for example, the surfaces of pharmaceutical tablets and the like using inkjet ink printing methods. The following description explains in detail the composition of an inkjet ink according to an embodiment of the present invention and a tablet including a printed part that is printed using the inkjet ink.

### [Composition of inkjet ink]

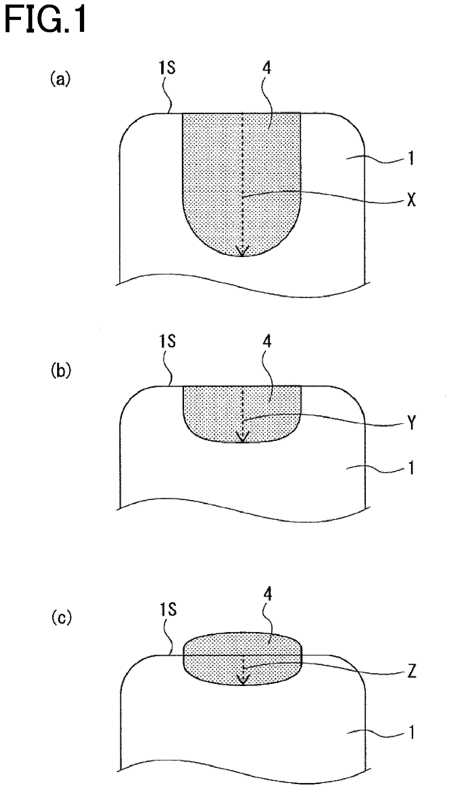

The inkjet ink according to the present embodiment is an edible inkjet ink and contains at least one of Food Red No. 102 and Food Blue No. 1 as a food dye (food pigment). Hereinafter, these food dyes are simply termed Red No. 102 and Blue No. 1. The inkjet ink according to the present embodiment contains a saccharide whose solubility in water satisfies predetermined conditions, as a fixing agent for fixing the ink to the surface of a printing target (e.g., solid formulation). Specifically, in the present embodiment, a disaccharide whose solubility in 100 ml of water at 20°C is less than 20 g is used as a fixing agent. With this composition, photofading of printed images can be sufficiently suppressed, and lightfastness of the inkjet ink can be improved. Referring to Fig. 1, a description in this regard will be given below.

Fig. 1 is a set of schematic diagrams illustrating a relationship between fixation of ink and lightfastness on the surface of a printing target (solid formulation). The solid formulation used herein is, for example, a tablet provided with a film-coated part having few voids, i.e., a film-coated tablet. It should be noted that the present invention should not be limited to this, but an uncoated tablet or capsule tablet may be used as a printing target.

Figs. 1(a) to 1(c) are schematic cross-sectional views in the thickness direction, illustrating the states of tablets (film-coated tablets herein) on which inkjet inks A to C with different compositions are printed and left to stand for a predetermined period of time. In the present examples, the inkjet ink A is an ink using a specific dye 4 as a colorant, and water as a solvent. The specific dye 4 may only need to contain either one of Red No. 102 and Blue No. 1 but, herein, Red No. 102 is assumed to be contained as an example. It should be noted that the following explanation regarding Fig. 1 is also applied to the case where the specific dye 4 contains Blue No. 1 and the case where it contains both of Red No. 102 and Blue No. 1.

The inkjet ink B is an ink obtained by adding a predetermined saccharide to the inkjet ink A. The saccharide contained in the inkjet ink B is a saccharide that has a relatively high solubility in water (e.g., maltose) and does not correspond to the fixing agent of the present embodiment. The inkjet ink C is an ink obtained by adding a disaccharide corresponding to the above fixing agent to the inkjet ink A.

Specifically, Fig. 1(a) is a schematic cross-sectional view in the thickness direction illustrating the state of a tablet where the inkjet ink A containing the specific dye 4 as a colorant and water as a solvent is printed on a surface 1S of a substrate 1 of the tablet and left to stand for a predetermined period of time (e.g., 140 hours). Fig. 1(b) is a cross-sectional view in the thickness direction illustrating the state of a tablet where the inkjet ink B is printed on the surface 1S of the substrate 1 of the tablet and left to stand for a predetermined period of time (e.g., 140 hours). Fig. 1(c) is a cross-sectional view in the thickness direction illustrating the state of a tablet where the inkjet ink C is printed on the surface 1S of the substrate 1 of the tablet and left to stand for a predetermined period of time (e.g., 140 hours).

First, taking the inkjet inks A and B as examples, the presence or absence of saccharides in the inkjet inks and permeability of dyes will be described.

Among various colorants used for inkjet inks, tar pigments, for example, used as food dyes can cause discoloration in the characters, images, and the like printed on the surfaces such as of solid formulations. For example, Red No. 102 and Blue No. 1 among the tar pigments have caused discoloration of printed images due to permeation into solid formulations or photolysis over time. In this regard, it is known that addition of saccharides to inkjet inks can suppress permeation of dyes into the solid formulations and suppress discoloration of the printed images.

As shown in Fig. 1(a), if the inkjet ink A containing no saccharides is printed, the specific dye 4 permeates into the substrate 1 of the tablet after the lapse of a predetermined time from printing. Herein, the depth of permeation of the specific dye 4 in the inkjet ink A after the lapse of the predetermined time is expressed as a permeation depth X (µm). In contrast, as shown in Fig. 1(b), if the inkjet ink B containing the saccharide is printed, a permeation depth Y (µm) of the inkjet ink B after the lapse of the predetermined time from printing is smaller than the permeation depth X of the inkjet ink A (X>Y).

In the inkjet ink B, viscosity of the ink increases, for example, due to addition of the predetermined saccharide, and permeation of the specific dye 4 into the substrate 1 of the tablet is suppressed. Therefore, if the inkjet ink B is printed, the specific dye 4 remains in the vicinity of the surface 1S of the substrate 1 of the tablet even after the lapse of the predetermined time from printing. In other words, inkjet inks containing predetermined saccharides can suppress discoloration of the printed images caused by permeation of dyes (e.g., Red No. 102 and/or Blue No. 1), compared to the case where no saccharides are added. However, the inkjet ink B to which the predetermined saccharide has been added cannot sufficiently suppress discoloration of the printed image caused by light irradiation (photofading).

In this regard, the inkjet ink according to the present embodiment can fix the ink on the surfaces of printing targets and sufficiently suppress photofading of the printed images to improve lightfastness, by selecting saccharides and types or physical properties of the solvent components added. The present inventors have found that it is effective to fix the ink on the surface of a printing target (e.g., tablet) in order to suppress photofading of the printed image. In the present example, the ink is fixed to the surface 1S of the tablet to thereby fix the specific dye 4 to the surface 1S, so that, resultantly, the specific dye 4 can remain on the surface 1S at a high density.

High density of the specific dye 4 on the surface 1S of the substrate 1 of the tablet can decrease the proportion of the colorant in the specific dye 4 on the surface 1S, for example, causing photofading due to photolysis. Specifically, even when photofading occurs in part of the specific dye 4, the influence is limited, and deterioration in visibility (readability) of the printed image can be suppressed. Thus, visibility of the printed image as a whole is maintained and, as a result, photofading of the printed image can be sufficiently suppressed. Accordingly, lightfastness of the inkjet ink can be improved. Furthermore, the present inventors have found that, among saccharides, disaccharides with relatively low solubility in have a function of fixing inks, which contain Red No. 102 and/or Blue No. 1 as colorants, to the surfaces of printing targets.

Specifically, the inkjet ink according to the present embodiment contains at least one of Red No. 102 and Blue No. 1, which is a colorant, as a food dye, and also contains a disaccharide whose solubility in 100 ml of water at 20°C is less than 20 g, as a fixing agent. With this composition, the ink containing a specific tar pigment used as a colorant (Red No. 1 02 or Blue No. 1 in the present example) can be fixed to the surface of a printing target, and the specific tar pigment can remain at the surface at a high density. Thus, the inkjet ink of the present embodiment can sufficiently suppress photofading of printed images and improve lightfastness.

As shown in Fig. 1(c), if the inkjet ink C corresponding to the inkjet ink of the present embodiment is printed on the surface 1S of the substrate 1 of the tablet, the specific dye 4 (Red No. 102 herein) is fixed to the surface 1S. Specifically, if the inkjet ink C is printed, a permeation depth Z (µm) of the specific dye 4 after the lapse of a predetermined time from printing further decreases compared to the permeation depth Y of the specific dye 4 in the inkjet ink B containing the predetermined saccharide (Y>Z). For example, the permeation depth Z of the specific dye 4 in the inkjet ink C decreases to about 50% of the permeation depth Y of the specific dye 4 in the inkjet ink B. In other words, the inkjet ink C can further suppress permeation of the specific dye 4. Therefore, the specific dye 4 remains on the surface 1S at a high density to suppress deterioration in visibility of the printed image as described above. Thus, while photofading of the printed image can be sufficiently suppressed, the inkjet ink C can improve lightfastness.

The inkjet ink B allows the specific dye 4 to remain in the substrate 1 of the tablet in the vicinity of the surface 1S; however, permeation of the specific dye 4 cannot be sufficiently suppressed and thus the ink cannot be fixed to the surface 1S. In other words, since the density of the specific dye 4 remaining on the surface 1S is low in the inkjet ink B, photofading of the printed image cannot be sufficiently suppressed and thus lightfastness is not improved.

Thus, with the disaccharide added as a fixing agent, the inkjet ink of the present embodiment can significantly suppress permeation of the specific tar pigment (Red No. 102 or Blue No. 1) into the printing target to fix the ink to the surface of the printing target. Thus, the inkjet ink of the present embodiment can suppress photofading of the printed image to improve lightfastness and, furthermore, can suppress discoloration of the printed image due to permeation of the specific tar pigment into the printing target.

As shown in Fig. 1(c), the inkjet ink C can be fixed to the surface 1S of the substrate 1 of the tablet with a slight rise. Thus, if the inkjet ink C is printed, for example, the print is perceived as dark and floating on the surface 1S. Therefore, the inkjet ink C can improve visibility of the printed image when printed, compared to the inkjet inks A and B, for example. In other words, in addition to reducing deterioration in visibility of the printed image due to photofading, the inkjet ink of the present embodiment can impart good visibility to the printed image when printed.

Hereinafter, lightfastness of the inkjet ink of the present embodiment will be described in detail.

The inkjet ink of the present embodiment may only need to have a color difference ΔE of 15 or less according to JIS Z 8781 before and after lightfastness testing. The lightfastness testing herein refers to, for example, a test of comparing values of the color difference ΔE (based on JIS Z 8781), i.e., variation in chromaticity and optical color density, before and after visible light irradiation, for the printed image which is printed using the inkjet ink of the present embodiment. Specifically, the color difference ΔE is measured before and after irradiation of a cumulative 1.2 million lux of visible light to the printed image and the values are compared to each other. For the visible light irradiation, a Xenon Weather-Ometer (Ci4000 manufactured by Toyo Seiki Seisaku-sho, Ltd.) is used, for example. The printed image used in lightfastness testing is an image which is solid-printed on a tablet (e.g., film-coated tablet), for example, as a printing target.

The necessary and sufficient lightfastness to be achieved as a result of forming a printed image on a pharmaceutical tablet, etc. substantially corresponds to the color difference ΔE of a printed image being 15 or less before and after visible light irradiation in lightfastness testing of applying visible light of 1.2 million lux. Use of the color difference ΔE of 15 or less as a criterion of sufficient lightfastness has been derived as a result of opinion tests conducted by the present inventors with medical professionals. Specifically, when the color difference ΔE exceeds 15, visibility of the printed image (e.g., readability of the print) begins to be perceived as decreased. In other words, if the color difference ΔE is 15 or less before and after visible light irradiation, photofading can be sufficiently suppressed, and it can be said that the inkjet ink has good lightfastness. This numerical value, which is larger than the standard color difference ΔE of 3 to 6 for general commercial printed matter, was obtained in view of the fact that, usually, a tablet before exposure and the tablet after exposure are assumed not to be compared to each other, and the printed image on the tablet surface is assumed to naturally fade.

The reason why a disaccharide satisfying the condition of solubility being less than 20 g in 100 ml of water at 20°C is used as a fixing agent in the inkjet ink of the present embodiment is that, if a saccharide not satisfying the above condition (e.g., maltose with a solubility of 52 g in 100 ml of water at 20°C) is added, the specific tar pigment, i.e., Red No. 102 or Blue No. 1, on the tablet surface is not necessarily sufficiently fixed to the surface of the printing target and the color difference ΔE may exceed 15 if lightfastness testing is conducted for the solid-printed image. With a disaccharide whose solubility in water satisfies the above condition added as a fixing agent, the color difference ΔE comes to be less than 15 before and after lightfastness testing, and good lightfastness is imparted to the inkjet ink.

Hereinafter, components composing the inkjet ink of the present embodiment will be described.

### (Colorant)

As described above, the inkjet ink of the present embodiment contains at least one of Red No. 102 and Blue No. 1 as a food dye. Red No. 102 and Blue No. 1 are tar pigments, of which Red No. 102 is also referred to as New Coccine. Blue No. 1 is also referred to as Brilliant Blue FCF. The inkjet ink of the present embodiment may contain both or either of Red No. 102 and Blue No. 1.

The blending ratio of food dyes including the specific tar pigments (Red No. 102 and/or Blue No. 1), i.e., the total content of the food dyes, in the inkjet ink of the present embodiment is preferred to be in the range of 1 mass% or more and 15 mass% or less with respect to the total mass of the ink. With this composition, good visibility can be imparted to the printed image and high intermittent resumability can be imparted thereto. If the total content of food dyes is in the above range, addition of the fixing agent can more reliably allow the specific tar pigments to remain at the surface of the printed matter at a high density to suppress photofading of the printed image and reliably improve lightfastness of the inkjet ink of the present embodiment.

In contrast, if the total content of the food dyes is less than 1 mass%, the overall printed color tends to be lighter and thus visibility of the printed image tends to be deteriorated. If the content exceeds 15 mass%, the pigments in the ink may precipitate as solids or may settle due to deterioration in dissolution stability of the colorants, which could cause nozzle clogging of the inkjet head during printing and thus could deteriorate what is called print resumability (intermittent resumability).

It should be noted that the inkjet ink of the present embodiment may contain pigments (colorants) other than the specific tar pigments, i.e., Red No. 102 and Blue No. 1. There is no particular limitation in the pigments other than these two types, as long as they are edible. Pigments that can be added to the inkjet ink of the present embodiment can be appropriately selected from, for example, known synthetic and natural food pigments. In the case where the inkjet ink of the present embodiment contains colorants other than the specific tar pigments as food dyes, the total content of the food dyes is also preferred to be in the above range (1 mass% or more and 15 mass% or less). Thus, good visibility can be imparted to the printed image, while lightfastness of the inkjet ink is improved, and sufficient intermittent resumability can be imparted to the inkjet ink.

The synthetic food pigments include, for example, tar pigments, natural pigment derivatives, natural synthetic pigments, and the like. Examples of the tar pigments include Food Red No. 2, Food Red No. 3, Food Red No. 40, Food Red No. 104, Food Red No. 105, Food Red No. 106, Food Yellow No. 4, Food Yellow No. 5, Food Blue No. 2, Food Red No. 2 Aluminum Lake, Food Red No. 3 Aluminum Lake, Food Red No. 40 Aluminum Lake, Food Yellow No. 4 Aluminum Lake, Food No. 5 Aluminum Lake, Food Blue No. 1 Aluminum Lake, and Food Blue No. 2 Aluminum Lake. The natural pigment derivatives include, for example, norbixin potassium. The natural synthetic pigments include, for example, β-carotene and riboflavin.

Examples of the natural food pigments include anthocyanin pigments, carotenoid pigments, quinone pigments, chlorophyll pigments, flavonoid pigments, betaine pigments, Monascus pigments, and other natural pigments originating from natural products. Examples of the anthocyanin pigments include red radish pigment, red cabbage pigment, red rice pigment, elderberry pigment, cowberry pigment, gooseberry pigment, cranberry pigment, salmon berry pigment, perilla pigment, sim blueberry pigment, strawberry pigment, dark sweet cherry pigment, cherry pigment, hibiscus pigment, huckleberry pigment, grape juice pigment, grape skin pigment, black currant pigment, blackberry pigment, blueberry pigment, plum pigment, whortleberry pigment, boysenberry pigment, and mulberry pigment, purple potato pigment, purple corn pigment, Chinese purple potato pigment, raspberry pigment, red currant pigment, loganberry pigment, and other anthocyanin pigments. Examples of the carotenoid pigments include annatto pigment, gardenia yellow pigment, and other carotenoid pigments. Examples of the quinone pigments include cochineal pigment, lithospermum root pigment, lac pigment, and other quinone pigments. Examples of the flavonoid pigments include safflower yellow pigment, kaoliang pigment, onion pigment, and other flavonoid pigments. Examples of the betaine pigments include beet red pigment. Examples of the Monascus pigments include Monascus purpureus pigment, and Monascus yellow pigment. Examples of other pigments originating from natural products include turmeric pigment, Trichotomine pigment, gardenia red pigment, and spirulina blue pigment.

### (Fixing agent)

As described above, the inkjet ink according to the present embodiment contains a fixing agent which fixes the ink containing the specific tar pigments (Red No. 102 and/or Blue No. 1) to the surface of a printing target. Specifically, the fixing agent contained in the inkjet ink of the present embodiment is a disaccharide whose solubility in 100 ml of water at 20°C is less than 20 g, e.g., lactose and cellobiose. Solubility of lactose in 100 ml of water at 20°C is 16 g. Solubility of cellobiose in 100 ml of water at 20°C is 14 g. Fixing agents with this composition can fix the ink containing the specific tar pigments to the surfaces of printing targets. Thus, the inkjet ink of the present embodiment can sufficiently suppress photofading of the printed image and improve lightfastness. The disaccharide (e.g., lactose or cellobiose) used as a fixing agent in the present embodiment can also suppress decomposition due to the inkjet ink being irradiated with light (due to photolysis). Therefore, the occurrence of photofading can also be suppressed.

The blending ratio of fixing agents in the inkjet ink of the present embodiment, i.e., the total content of the disaccharides (lactose and cellobiose), is preferred to be in the range of 0.5 mass% or more and 15 mass% or less with respect to the total mass of the ink. With this composition, the effect of fixing the ink is more reliably exerted by the disaccharide to reliably improve lightfastness of the inkjet ink. Furthermore, high intermittent resumability can be imparted to the inkjet ink of the present embodiment.

However, if the blending ratio of fixing agents is less than 0.5 mass%, the ink fixing effect can be reduced. If the blending ratio of fixing agents (lactose and/or cellobiose) exceeds 15 mass%, viscosity of the ink may increase or the fixing agents in the ink may precipitate as solids or may be settled due to deterioration in dissolution stability of the fixing agents, which could cause nozzle clogging of the inkjet head during printing and thus could deteriorate intermittent resumability.

### (Solvent)

In addition to the specific tar pigments (Red No. 102 and/or Blue No. 1) and the fixing agents, the inkjet ink of the present embodiment may contain a solvent (dispersion medium) to dissolve (disperse) the specific tar pigments and the fixing agents. The inkjet ink of the present embodiment may only need to contain at least one of water (e.g., purified water), ethanol, glycerin, propylene glycol, and isopropyl alcohol, as a solvent.

Saccharides are difficult to dissolve in alcohols. In this regard, in the present embodiment, the solvent contains any of ethanol, propylene glycol, and isopropyl alcohol, and therefore solubility, in the solvent, of the disaccharides used as fixing agents decreases. Thus, in the inkjet ink of the present embodiment, the ink fixing effect is further improved by the disaccharides, so that the specific tar pigments (Red No. 102 and/or Blue No. 1 in the present example) contained in the ink can remain at the surfaces of printing targets (e.g., tablets) with higher density. Accordingly, lightfastness of the inkjet ink of the present embodiment can be further improved.

The propylene glycol can function as a wetting agent to prevent the ink from drying at the inkjet nozzles and impart sufficient intermittent resumability to the ink. The ethanol or isopropyl alcohol has high volatility and thus can improve transfer resistance (dryness) of the inkjet ink. With the addition of the above alcohols to the solvent in addition to water, lightfastness of the inkjet ink can be further improved, and the inkjet ink can be imparted with functions according to the components.

The blending ratios of the components of the solvent should be unlimited in the inkjet ink of the present embodiment; however, if the solvent contains propylene glycol, the blending ratio of the propylene glycol, i.e., the amount of the propylene glycol added, may be in the range of 2 mass% or more and 40 mass% or less with respect to the total mass of the ink. With this composition, solubility of the fixing agents in the solvent reliably decreases, and the effect of fixing the ink containing the specific tar pigments is further improved. Therefore, better lightfastness can be imparted to the inkjet ink.

If the amount of the propylene glycol added is less than 10 mass%, the effect of decreasing solubility of the fixing agents in the solvent can be reduced. If the amount of the propylene glycol added exceeds 40 mass%, drying of the printed surface on the tablet surface may be delayed, and this may cause defects in which undried ink adheres to other tablets and causes staining when printed tablets are brought into contact with each other (poor transfer).

If the solvent contains ethanol, the blending ratio of the ethanol, i.e., the amount of the ethanol added, may be in the range of 6.5 mass% or more and 40 mass% or less with respect to the total mass of the ink. With this composition, solubility of the fixing agents in the solvent reliably decreases, and the effect of fixing the ink containing the specific tar pigments is further improved. Therefore, better lightfastness can be imparted to the inkjet ink.

If the amount of the ethanol added is less than 6.5 mass%, the effect of decreasing solubility of the fixing agents in the solvent can be reduced. If the amount of the ethanol added exceeds 40 mass%, the ink may dry at the inkjet nozzles, causing clogging at the nozzles of the inkjet head during printing, and this can deteriorate intermittent resumability.

### (Internal-sizing resin)

The inkjet ink of the present embodiment may contain an internal-sizing resin other than the above pigments and solvent. The internal-sizing resin that can be added to the inkjet ink of the present embodiment may be an edible resin-like material in the form of a water-soluble powder, paste, or flakes which are capable of forming a coating on the surface of a tablet when dried following printing. Examples of the internal-sizing resin include polyvinyl alcohols (PVAs), hydroxypropyl celluloses (HPCs), hydroxypropyl methylcelluloses (HPMCs), polyvinylpyrrolidones (PVPs), high molecular weight polyethylene glycols (PEGs) such as polyethylene glycol 4000 or polyethylene glycol 1540, shellac resins, methacrylic acid copolymers (product name: Eudragit S 100), maltodextrins, and erythritols.

### (Leveling agent)

The inkjet ink of the present embodiment may contain a leveling agent other than the above pigments, solvent, and internal-sizing resin. The levelling agent that can be added to the inkjet ink of the present embodiment may only need to be an edible and water-soluble surfactant. Examples of the levelling agent include polyglycerin fatty acid esters (e.g., Decaglyceryl distearate Q-182S or Decaglyceryl monolaurate Q-12S manufactured by Taiyo Kagaku Co.,Ltd.), sorbitan fatty acid esters (e.g., NIKKOLSL-10 manufactured by Nikko Chemicals Co., Ltd.), sucrose fatty acid esters (e.g., DK Ester F-110 manufactured by DKS Co. Ltd.), and polysorbates (Emazole S-120 series manufactured by Kao Corporation).

### (Printing method)

The printing method using the inkjet ink of the present embodiment is not particularly limited, but printing using an inkjet device, such as a commercially available inkjet printer, may be used. Therefore, the inkjet ink of the present embodiment has a wide range of applications and is very useful. For example, the inkjet ink of the present embodiment can be printed using a drop-on-demand inkjet device including a piezoelectric element (piezoelectric ceramic) as an actuator, or inkjet devices of other types.

Examples of the drop-on-demand inkjet device include a thermal-inkjet type device ejecting an inkjet ink using water vapor pressure generated by instantaneously heating a micro-heating element to a high temperature (of 200°C to 300°C), an electrostatic-type device ejecting an inkjet ink by electrostatically vibrating an actuator, and an ultrasonic-type device using an ultrasonic cavitation phenomenon. If the inkjet ink of the present embodiment has charging performance, a continuous injection-type device may be used.

### [Tablet]

The inkjet ink of the present embodiment may be used for printing characters or an image on the surfaces of tablets, for example, using the above printing methods. In other words, the tablet according to the present embodiment may have a printed part, i.e., a printed image, which is printed using the inkjet ink of the present embodiment. As long as the inkjet ink according to the present embodiment is used, lightfastness can be improved in the printed images (e.g., printed image 3), for example, provided on the surfaces of pharmaceutical tablets using inkjet printing methods. The following description explains a composition of a tablet having a printed image printed using the inkjet ink of the present embodiment.

The tablet of the present embodiment is, for example, a pharmaceutical tablet. Examples of the pharmaceutical tablet herein include film-coated tablets having an outermost surface on which a water-soluble surface layer is formed, as well as uncoated tablets (bare tablets), sugar-coated tablets, enteric tablets, and orally disintegrating tablets.

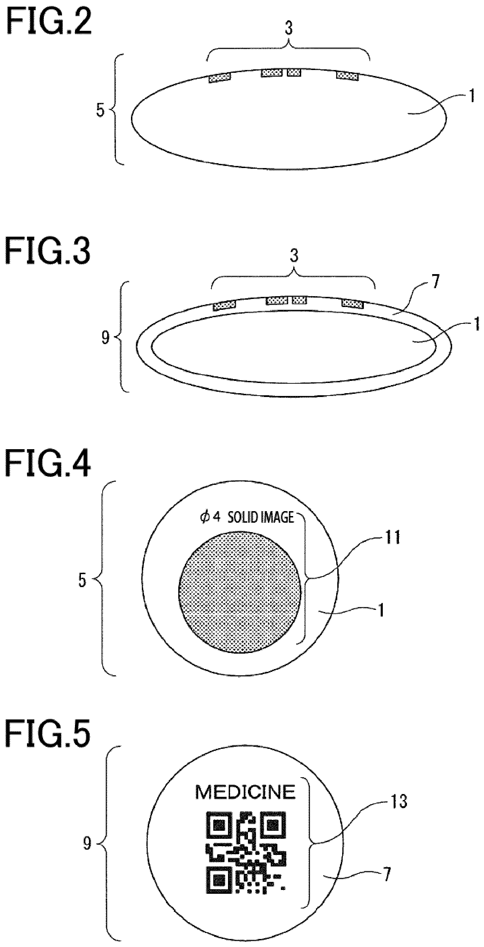

Fig. 2 is a schematic cross-sectional view illustrating an example of a pharmaceutical tablet (uncoated tablet) having a print (characters or image). Fig. 2 shows uncoated tablet printed matter 5, in cross-sectional view, with a printed image 3, such as characters, printed on the upper surface of a substrate 1 of the tablet.

Fig. 3 is a schematic cross-sectional view illustrating an example of a pharmaceutical tablet (film-coated tablet) provided with a print (characters or image) thereon. Fig. 3 shows film-coated tablet printed matter 9, in cross-sectional view, with a printed image 3, such as characters, printed on the upper surface of the substrate 1 of the tablet, on the surface of which a film coating layer 7 is formed.

In the present embodiment, a solid image may be printed as an uncoated tablet printed image 11 as shown in Fig. 4, or a two-dimensional barcode may be printed as a film-coated tablet printed image 13 as shown in Fig. 5.

The pharmaceutical table may contain unlimited active ingredients. Examples of the active ingredients include, but are not limited thereto, substances effective for preventing or treating various diseases (e.g., substances having a sleep-inducing effect, tranquilizer activity, antibacterial activity, antihypertensive effect, anti-angina activity, analgesic effect, antiinflammatory activity, tranquilizing effect, diabetes treatment activity, diuretic effect, anticholinergic activity, anti-hyperacidity effect, antiepileptic effect, ACE inhibitory activity, β-receptor antagonist or agonist activity, anesthetic action, appetite suppressant action, antiarrhythmic effect, antidepressant effect, anticoagulant activity, antidiarrheal effect, antihistamine activity, antimalarial effect, antitumor activity, immunosuppressive activity, antiparkinsonian effect, antipsychotic effect, antiplatelet activity, and antihyperlipidemic effect), substances having a scavenging effect, and substances having a scent or a deodorant action.

In the tablet of the present embodiment, carriers which are compatible from the perspective of usage with the active ingredients may be mixed as necessary. For example, pharmaceutical tablets may comprise carriers which are compatible from the pharmaceutical perspective. As carriers compatible from the pharmaceutical perspective, various organic or inorganic carriers, which are commonly used as pharmaceutical materials, may be appropriately mixed, such as excipients, lubricants, binders, disintegrants, and thickeners. As necessary, additives such as antiseptics, antioxidants, colorants, or sweeteners may be used.

Although the present embodiment has been described taking an example of a pharmaceutical tablet as a tablet, tablet is not limited to this tablet of the present invention. Printing targets of the inkjet ink of the present embodiment are not particularly limited. For example, the inkjet ink of the present embodiment may be printed on the surfaces of various tablets, including tablets to be administered to non-human animals (e.g., pets, livestock, poultry, etc.), or tablets of feed, fertilizers, and cleaning agents, and food tablets such as soda-pop flavored confectionery tablets and supplement tablets. The inkjet ink of the present embodiment does not particularly limit the size of the printing target, but may be applied to tablets of various sizes.

### (Advantageous effects of the present embodiment)

(1) The inkjet ink according to the present embodiment contains at least one of Red No. 102 and Blue No. 1 as a food dye, and also contains a disaccharide whose solubility in 100 ml of water at 20°C is less than 20 g, as a fixing agent.
   With this composition, photofading of printed images can be sufficiently suppressed and lightfastness of the inkjet ink can be improved, compared to the conventional art.
(2) The inkjet ink according to the present embodiment contains at least one of lactose and cellobiose as a fixing agent.
   With this composition, photofading of printed images can be sufficiently suppressed and good lightfastness can be imparted to the inkjet ink, compared to the conventional art.
(3) The inkjet ink according to the present embodiment may contain at least one of water, ethanol, glycerin, propylene glycol, and isopropyl alcohol, as a solvent.
   With this composition, the inkjet ink can be imparted with performance according to each component of the solvent (improvement in intermittent resumability or improvement in dryness of tablets), while further improving lightfastness of the ink, compared to the conventional art.
(4) In the inkjet ink according to the present embodiment, the blending ratio of food dyes may be in the range of 1 mass% or more and 15 mass% or less with respect to the total mass of the ink.
   With this composition, the inkjet ink can be imparted with high visibility and intermittent resumability, while improving lightfastness of the ink, compared to the conventional art.
(5) The blending ratio of the fixing agent contained in the inkjet ink according to the present embodiment may be in the range of 0.5 mass% or more and 15 mass% or less with respect to the total mass of the ink.
   With this composition, the inkjet ink can be imparted with intermittent resumability, while reliably improving lightfastness of the ink, compared to the conventional art.
(6) The tablet according to the present embodiment is provided with the printed image 3 (one example of the printed part) which is printed using the inkjet ink described above.
   With this configuration, photofading of the printed image 3, etc. directly printed on the surface, etc. of the tablet can be sufficiently suppressed and lightfastness of the printed image 3 can be sufficiently improved, compared to the conventional art. Furthermore, edibility can also be imparted to the printed image portions on the surface of the tablet.
(7) The tablet according to the present embodiment may be a pharmaceutical tablet.
   Using the inkjet ink according to the present embodiment, photofading can be sufficiently suppressed in pharmaceutical tablets, and the occurrence of incorrect dispensing or dosing can be reduced, which would occur due to deterioration in visibility of the printed images.

### [Examples]

The present invention will be further described in detail using examples; however the present invention should not be limited to these examples.

### <Example 1>

A procedure of preparing an inkjet ink of Example 1 will be described.

### (Production of inkjet ink)

First, a printing ink was prepared. Inkjet inks contain components such as a pigment (colorant), solvent, and fixing agent. First of all in the preparation procedure, a fixing agent was added to a solvent to obtain a transparent base liquid. Next, a food dye was added to the transparent base liquid. Thus, an ink of the present example was prepared. The individual components will be specifically described below.

In the present example, purified water (deionized water) was used as the solvent. Lactose as a fixing agent was added to the solvent and the mixture was stirred for about 1 hour to obtain a transparent base liquid. Red No. 102 that was a food dye as a colorant was added to the transparent base liquid to obtain an inkjet ink of Example 1. The blending ratio of Red No. 102 as a colorant was 5.0 mass%, that of purified water as a solvent was 90.0 mass%, and that of lactose as a fixing agent was 5.0%, with respect to the total mass of the inkjet ink of Example 1.

### <Example 2>

As a solvent, purified water and ethanol were used. Specifically, ethanol was added to purified water and the mixture was stirred well to obtain a solvent (mixed solvent). In the solvent, the blending ratio of purified water was 78.0 mass% and that of ethanol was 12.0 mass%, with respect to the total mass of the ink. Except for these points, an inkjet ink of Example 2 was obtained as in Example 1.

### <Example 3>

Purified water and isopropyl alcohol were mixed to obtain a solvent (mixed solvent). In the solvent, the blending ratio of isopropyl alcohol was 12.0 mass% with respect to the total mass of the ink. Except for these points, an inkjet ink of Example 3 was obtained as in Example 2.

### <Example 4>

Purified water, glycerin, propylene glycol, and isopropyl alcohol were mixed to obtain a solvent (mixed solvent). In the solvent, the blending ratio of purified water was 52.0 mass%, that of glycerin was 10.0 mass%, that of propylene glycol was 20.0 mass%, and that of isopropyl alcohol was 8.0%, with respect to the total mass of the ink. Except for these points, an inkjet ink of Example 4 was obtained as in Example 1.

### <Example 5>

Purified water and ethanol were mixed to obtain a solvent (mixed solvent). In the solvent, the blending ratio of purified water was 30.0 mass% and that ethanol was 60.0 mass%, with respect to the total mass of the ink. Except for these points, an inkjet ink of Example 5 was obtained as in Example 1.

### <Example 6>

Purified water and isopropyl alcohol were mixed to obtain a solvent (mixed solvent). In the solvent, the blending ratio of purified water was 30.0 mass% and that of isopropyl alcohol was 60.0 mass%, with respect to the total mass of the ink. Except for these points, an inkjet ink of Example 6 was obtained as in Example 1.

### <Example 7>

Purified water, propylene glycol, and isopropyl alcohol were mixed to obtain a solvent (mixed solvent). In the solvent, the blending ratio of purified water was 80.5 mass%, that of propylene glycol was 1.5 mass%, and that of isopropyl alcohol was 12.0%, with respect to the total mass of the ink. The blending ratio of lactose as a fixing agent was 1.0 mass% with respect to the total mass of the ink. Except for these points, an inkjet ink of Example 7 was obtained as in Example 1.

### <Example 8>

The components of the solvent (mixed solvent) were the same as in Example 7, but the blending ratio of purified water was 76.5 mass% with respect to the total mass of the ink. Except for these points, an inkjet ink of Example 8 was obtained as in Example 1.

### <Example 9>

The components of the solvent (mixed solvent) were the same as in Example 7, but the blending ratio of purified water was 71.5 mass% with respect to the total mass of the ink. The blending ratio of lactose as a fixing agent was 10.0 mass% with respect to the total mass of the ink. Except for these points, an inkjet ink of Example 9 was obtained as in Example 1.

### <Example 10>

The components of the solvent (mixed solvent) were the same as in Example 7, but the blending ratio of purified water was 66.5 mass% with respect to the total mass of the ink. The blending ratio of lactose as a fixing agent was 15.0 mass% with respect to the total mass of the ink. Except for these points, an inkjet ink of Example 10 was obtained as in Example 1.

### <Example 11>

The blending ratio of Red No. 102 as a colorant was 1.0 mass% with respect to the total mass of the ink. The components of the solvent (mixed solvent) were the same as in Example 7, but the blending ratio of purified water was 80.5 mass% with respect to the total mass of the ink. Except for these points, an inkjet ink of Example 11 was obtained as in Example 1.

### <Example 12>

The blending ratio of Red No. 102 was 7.0 mass%, that of Blue No. 1 was 8.0 mass%, and that of colorants in total was 15.0 mass%, with respect to the total mass of the ink. The components of the solvent (mixed solvent) were the same as in Example 7, but the blending ratio of purified water was 66.5 mass% with respect to the total mass of the ink. Except for these points, an inkjet ink of Example 12 was obtained as in Example 1.

### <Example 13>

Blue No. 1 was used as a colorant at a blending ratio of 8.0 mass% with respect to the total mass of the ink. The components of the solvent (mixed solvent) were the same as in Example 7, but the blending ratio of purified water was 78.0 mass% with respect to the total mass of the ink. The blending ratio of lactose as a fixing agent was 0.5 mass% with respect to the total mass of the ink. Except for these points, an inkjet ink of Example 13 was obtained as in Example 1.

### <Example 14>

The components of the solvent (mixed solvent) were the same as in Example 7, but the blending ratio of purified water was 73.5 mass% with respect to the total mass of the ink. The blending ratio of lactose as a fixing agent was 5.0 mass% with respect to the total mass of the ink. Except for these points, an inkjet ink of Example 14 was obtained as in Example 13.

### <Example 15>

The components of the solvent (mixed solvent) were the same as in Example 7, but the blending ratio of purified water was 63.5 mass% with respect to the total mass of the ink. The blending ratio of lactose as a fixing agent was 15.0 mass% with respect to the total mass of the ink. Except for these points, an inkjet ink of Example 15 was obtained as in Example 13.

### <Example 16>

An inkjet ink of Example 16 was obtained as in Example 9 except that cellobiose was added as a fixing agent at a blending ratio of 10.0 mass% with respect to the total mass of the ink.

### <Example 17>

An inkjet ink of Example 17 was obtained as in Example 15 except that cellobiose was added as a fixing agent at a blending ratio of 15.0 mass% with respect to the total mass of the ink.

### <Example 18>

The components of the solvent (mixed solvent) were the same as in Example 7, but the blending ratio of purified water was 81.0 mass% with respect to the total mass of the ink. Cellobiose was added as a fixing agent at a blending ratio of 0.5 mass% with respect to the total mass of the ink. Except for these points, an inkjet ink of Example 18 was obtained as in Example 16.

### <Comparative Example 1>

An inkjet ink of Comparative Example 1 was obtained as in Example 18 except that no saccharides were added and the blending ratio of purified water in the solvent was 81.5 mass% with respect to the total mass of the ink.

### <Comparative Example 2>

Reduced isomaltulose whose solubility in 100 ml of water at 20°C was 38 g was added at a blending ratio of 10.0 mass% with respect to the total mass of the ink. Except for these points, an inkjet ink of Comparative Example 2 was obtained as in Example 9.

### <Comparative Example 3>

Maltose whose solubility in 100 ml of water at 20°C was 52 g was added as a fixing agent at a blending ratio of 10.0 mass% with respect to the total mass of the ink. Except for these points, an inkjet ink of Comparative Example 3 was obtained as in Example 9.

### <Comparative Example 4>

Red No. 3 was used as a colorant at a blending ratio of 5.0 mass% with respect to the total mass of the ink. Except for these points, an inkjet ink of Comparative Example 4 was obtained as in Comparative Example 1.

### <Comparative Example 5>

Red No. 3 was used as a colorant at a blending ratio of 5.0 mass% with respect to the total mass of the ink. Except for these points, an inkjet ink of Comparative Example 5 was obtained as in Example 8.

Each of the inks of Examples 1 to 18 and Comparative Examples 1 to 5 was passed through a membrane filter to remove solid foreign matter from the liquid. Specifically, each of the inks was passed once through a membrane filter (cellulose acetate film) having a pore size of 5.0 µm, and subsequently, was passed once through a membrane filter (cellulose acetate film) having a pore size of 0.8 µm to obtain a purified ink.

### <Evaluation>

Lightfastness, intermittent resumability, and tablet dryness of the purified inks obtained from the above examples and comparative examples were evaluated using the following method. The results of evaluation are shown in the following Tables 1 and 2 together with the ink compositions of the examples and comparative examples.

### (Lightfastness testing)

Using a piezoelectric ceramic-driven drop-on-demand inkjet head having a print resolution of 600 dpi in the main scanning direction and 600 dpi in the sub-scanning direction (conveyance direction of the recording medium such as a tablet) and having 2,656 nozzles in total, an image was printed on the following tablet using the inks of Examples 1 to 18 and Comparative Examples 1 to 5 at 6 pl per drop.

The tablet as a printing target was a film-coated tablet for testing (base: conditioned starch, coating agent: mixture of 70% hydroxypropyl methylcellulose and 30% titanium oxide, diameter: 6.5 mm). The printed image was a circular solid image (diameter: 4.0 mm). Thus, film-coated tablet printed matter was obtained.

Using a xenon weather meter (Ci4000 manufactured by Toyo Seiki Seisaku-sho, Ltd.), a cumulative 1.2 million lux of visible light was applied to the above film-coated tablet printed matter of the examples and comparative examples of which the chromaticity and optical color density were measured. Using a spectrophotometer, chromaticity and optical color density were measured for the film-coated tablet printed matter irradiated with visible light, and a comparison was made between values of the color difference ΔE (based on JIS Z 8781), i.e., variation in chromaticity and optical color density, before and after the visible light irradiation. The comparison results are shown in Tables 1 and 2. As described above, regarding the change of color before and after visible light irradiation, the present inventors have found that if the color difference ΔE according to JIS Z 8781 was 15 or less (ΔE≤15), photofading was sufficiently suppressed and the ink had good lightfastness. Thus, if ΔE≤15 was satisfied, the inkjet ink was determined to have good lightfastness and evaluated to be above the pass level.

### (Intermittent resumability testing)

Using a piezoelectric ceramic-driven drop-on-demand inkjet head having a print resolution of 600 dpi in the main scanning direction and 600 dpi in the sub-scanning direction (conveyance direction of the recording medium such as a tablet) and having 2,656 nozzles in total, each ink was left to stand for a specified period of time (15 min to 60 min) without flushing and then a test pattern was printed using the ink at 6 pl per drop, and it was confirmed that ink could be ejected from all the nozzles without ejection failure. In Tables 1 and 2, a standing time after which the ink could still be ejected was measured as an evaluation for intermittent resumability. The evaluation criteria were as follows.
Very good: 60 min or more and less than 120 min
Good: 30 min or more and less than 60 min
Fair: 15 min or more and less than 30 min
Poor: Less than 15 min

### (Tablet dryness (transfer resistance) testing)

Using a piezoelectric ceramic-driven drop-on-demand inkjet head having a print resolution of 600 dpi in the main scanning direction and 600 dpi in the sub-scanning direction (conveyance direction of the recording medium such as a tablet) and having 2,656 nozzles in total, an image was printed on the following tablet using the inkjet inks of the examples and comparative examples at 10 pl per drop.

10 seconds after printing, white copying paper pasted on a digital force gauge was brought into contact with the tablet subjected to printing, for 0.4 to 0.5 seconds at a pressure of 4 to 5 N to confirm that the printed ink was not transferred. In Tables 1 and 2, density of the transferred ink was visually observed as an evaluation for transfer resistance (dryness). The evaluation criteria were as follows.
Very good: There was no ink transfer.
Good: Ink was slightly transferred but difficult to visually recognize.
Poor: Ink was densely transferred.

The inkjet ink composition and the results of evaluation of the examples are shown in Table 1. The inkjet ink composition and the results of evaluation of the comparative examples are shown in Table 2. In Tables 1 and 2, blank cells indicate that the substances were not used. In Tables 1 and 2, the film-coated tablet is abbreviated as FC tablet. In Tables 1 and 2, solubility in 100 ml of water at 20°C is abbreviated as solubility.

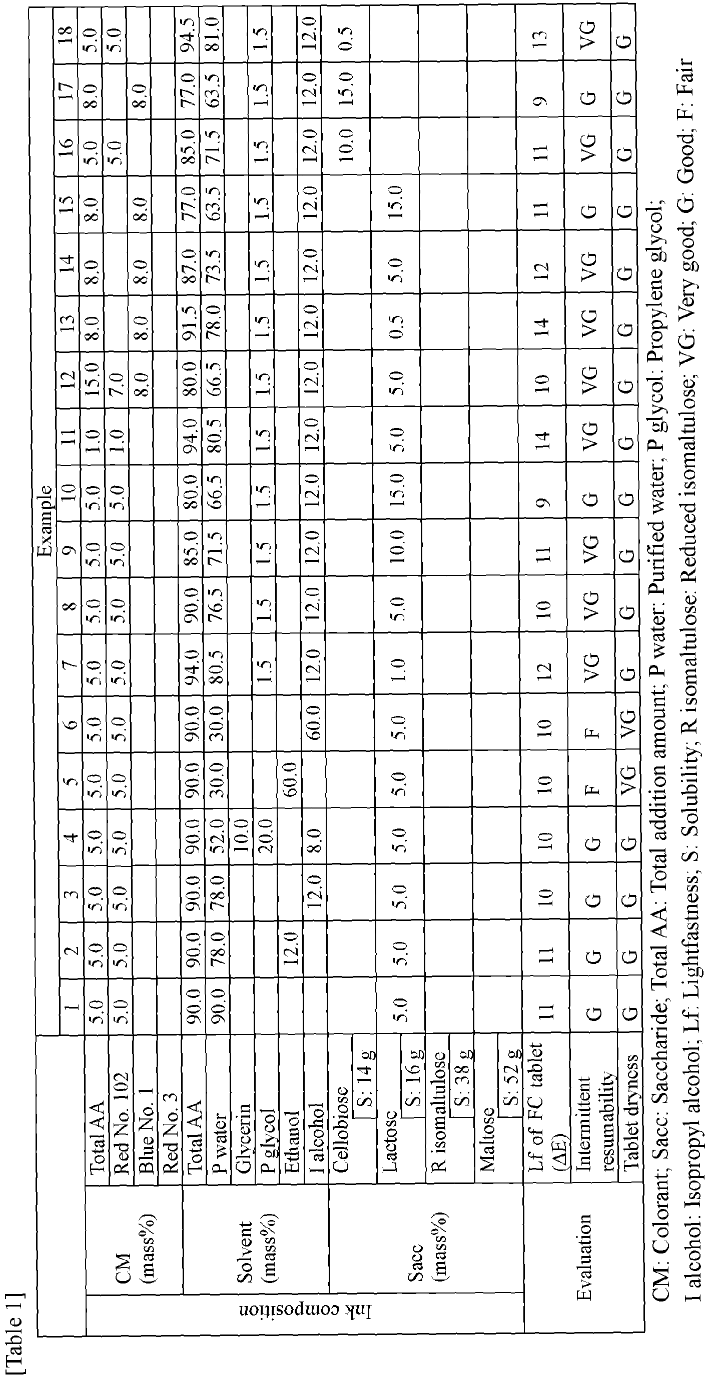

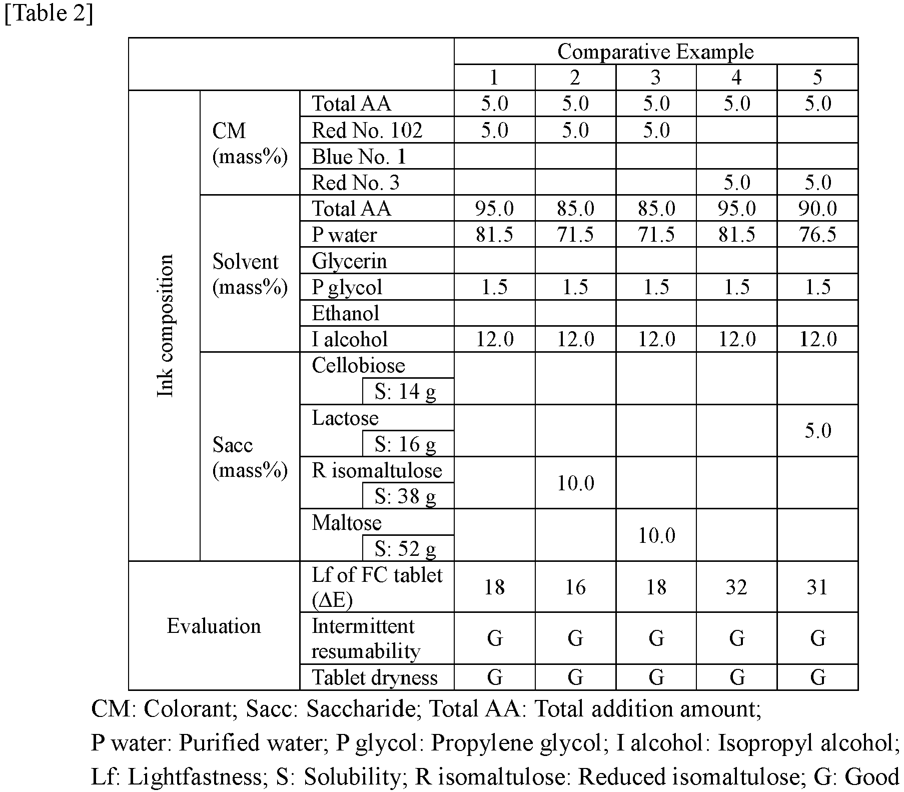

As shown in Table 1, the film-coated tablet printed matter of Examples 1 to 18 all showed the color difference ΔE of 15 or less (ΔE≤15) before and after visible light irradiation, in lightfastness testing. Specifically, the film-coated tablet printed matter of Examples 1 to 18 all showed the color difference ΔE in the range of 9 to 14, i.e., less than 15, before and after visible light irradiation. In other words, the inkjet inks of Examples 1 to 18 were found to sufficiently suppress photofading of the printed image and be imparted with good lightfastness. In contrast, as shown in Table 2, the film-coated tablet printed matter of Comparative Examples 1 to 5 all showed the color difference ΔE exceeding 15 (ΔE>15) before and after visible light irradiation, in lightfastness testing. In other words, the inkjet ink of Comparative Examples 1 to 5 were found not to sufficiently suppress photofading of the printed image and be insufficient in lightfastness.

In lightfastness testing, it was found that, if the inkjet ink contained at least one of Red No. 102 and Blue No. 1 as a food dye, and a disaccharide (e.g., cellobiose or lactose) having a solubility of 20 g or less in 100 ml of water at 20°C as a fixing agent, photofading of the printed image could be sufficiently suppressed and good lightfastness could be imparted to the printed image.

Furthermore, as shown in Table 1, the inkjet inks of Examples 1 to 18 were all Fair or had higher evaluation in intermittent resumability, and were found to have good lightfastness and sufficient intermittent resumability. In particular, although the blending ratio of the saccharide as a fixing agent was the upper limit (15 mass%) in the inkjet inks of Examples 10, 15 and 17, and the blending ratio of the colorant was the upper limit (15 mass%) in Example 12, they were found to have better intermittent resumability due to addition of the propylene glycol functioning as a wetting agent.

Furthermore, as shown in Table 1, the inkjet inks of Examples 1 to 18 were all Good or had higher evaluation in transfer resistance (tablet dryness), and were found to have good tablet dryness as well as lightfastness. In particular, the inkjet inks of Examples 5 and 6 were found to be imparted with very good tablet dryness due to the high blending ratio of ethanol or isopropyl alcohol.

The scope of the present invention should not be construed as being limited to the illustrated and described exemplary embodiments, but should encompass all embodiments that achieve effects equivalent to the intended effects of the present invention. Furthermore, the scope of the present invention should not be construed as being limited to combinations of the features of the invention defined by the claims, but could be defined by any desired combinations of specific features among all the features disclosed.

### [Reference Signs List]

- 1: Substrate of tablet
- 1S: Surface
- 3: Printed image
- 4: Specific dye
- 5: Uncoated tablet printed matter
- 7: Film coating layer
- 9: Film-coated tablet printed matter
- 11: Uncoated tablet printed image (solid image)
- 13: Film-coated tablet printed image (two-dimensional barcode)

## Claims

1. An inkjet ink comprising:
at least one of Red No. 102 and Blue No. 1 as a food dye; and
a disaccharide whose solubility in 100 ml of water at 20°C is less than 20 g, as a fixing agent.

2. The inkjet ink according to claim 1 comprising at least one of lactose and cellobiose as a fixing agent.

3. The inkjet ink according to claim 1 or 2 comprising:
at least one of water, ethanol, glycerin, propylene glycol, and isopropyl alcohol, as a solvent.

4. The inkjet ink according to any one of claims 1 to 3, wherein:
a blending ratio of the food dye is in a range of 1 mass% or more and 15 mass% or less with respect to a total mass of the ink.

5. The inkjet ink according to any one of claims 1 to 4, wherein:
a blending ratio of the fixing agent is in a range of 0.5 mass% or more and 15 mass% or less with respect to a total mass of the ink.

6. A tablet comprising a printed part that is printed using the inkjet ink according to any one of claims 1 to 5.

7. The tablet according to claim 6, wherein the tablet is a pharmaceutical tablet.
